# FASCICULE DE BREVET EUROPEEN

(11) **EP 1 250 118 B1**
(45) Date de publication et mention de la délivrance du brevet: **27.02.2008**
(21) Numéro de dépôt: 01903993.2
(22) Date de dépôt: 29.01.2001
(51) Int. Cl.: A61Q 5/00, A61K 8/46, A61K 8/73, A61Q 19/00

(54) **COMPOSITIONS COSMETIQUES COMPRENANT UN MELANGE POLYMERE CATIONIQUE/TENSIOACTIF ANIONIQUE ET UTILISATION DUDIT MELANGE COMME AGENT STRUCTURANT**
KOSMETISCHE ZUSAMMENSETZUNGEN ENTHALTEND EINE MISCHUNG VON KATIONISCHEN POLYMEREN/ANIONISCHEN TENSIDEN UND VERWENDUNG DIESER MISCHUNG ALS STRUKTURIERMITTEL
COSMETIC COMPOSITIONS COMPRISING A CATIONIC POLYMER/ANIONIC SURFACTANT MIXTURE AND USE OF SAID MIXTURE AS CONDITIONING AGENT

(30) Priorité: 28.01.2000 FR 0001134
(43) Date de publication de la demande: 23.10.2002
(73) Titulaire: RHODIA CHIMIE, 92512 Boulogne-Billancourt Cedex (FR)
(72) Inventeur: RICCA, Jean-Marc, Princeton,NJ 08542 (FR)
(74) Mandataire: Boittiaux, Vincent
(86) Numéro de dépôt international: PCT/FR2001/000275
(87) Numéro de publication internationale: WO 2001/054655

(56) Documents cités:
- WO-A-00/64411
- WO-A-98/00486
- WO-A-98/29094
- GB-A- 2 109 832
- US-A- 3 816 616
- US-A- 5 059 414
- E. D. GODDARD ET AL: "Protection of skin by cationic cellulosics: In-vitro testing methods" COSMETICS & TOILETRIES, vol. 97, no. 7, 1982, pages 55-69, XP000972389 WHEATON, IL, US ISSN: 0361-4387
- CHEMICAL ABSTRACTS, vol. 88, no. 16, 17 avril 1978 (1978-04-17) Columbus, Ohio, US; abstract no. 110373y, R. B. HANNAN ET AL: "Desorption of a cationic polymer from human hair : surfactant and salt effects" page 321; XP002155675 & TEXT. RES. J., vol. 48, no. 1, 1978, pages 57-58,
- HOFFMANN H ET AL: "GELS FROM MODIFIED HYDROXYETHYL CELLULOSE AND IONIC SURFACTANTS" POLYMER GELS AND NETWORKS,ELSEVIER, AMSTERDAM,NL, vol. 4, no. 5/06, 1996, pages 509-526, XP000972388 ISSN: 0966-7822

## Description

La présente invention a pour objet des compositions cosmétiques aqueuses comprenant un mélange d'au moins un polymère cationique et d'au moins un tensioactif anionique, de même que l'utilisation de ce mélange en tant qu'agent structurant dans des compositions cosmétiques aqueuses, plus particulièrement destinées au cheveu et/ou à la peau.

Les produits cosmétiques destinés à traiter le cheveu ou la peau, ou plus généralement les matières kératiniques, nécessitent pour une application, l'obtention d'une rhéologie particulière. De nombreuses technologies ont été développées pour conduire aux propriétés rhéologiques souhaitées, mais elles sont généralement peu satisfaisantes d'un point de vue sensoriel.

Parmi ces technologies, on peut citer les dérivés non ioniques de cellulose comme les hydroxyéthyl- ou hydroxypropyl- celluloses.

Récemment, des polymères associatifs en présence de tensioactifs ont été développés. Le greffage de chaînes alkyle sur des polymères hydrosolubles, confère à ces derniers des propriétés associatives en présence de tensioactifs anioniques, conduisant à des compositions dont le profil rhéologique est satisfaisant. Cependant, la préparation de tels composés s'avère difficile et économiquement peu viable. De plus, ces composés ne contribuent pas ou peu aux propriétés cosmétiques des compositions.

Par ailleurs le document Hoffman H et al "Gels from Modified Hydroxyethyl Cellulose and Ionic Surfactants," Polymer Gels and Networks, vol. 4 n° 5/06, 1996, pp 509-526, décrit des interactions avec des tensioactifs anioniques.

Il a été trouvé de façon inattendue, que certains polymères cationiques présentaient un caractère associatif en présence de tensioactifs anioniques, conduisant à l'obtention de compositions présentant de nouvelles textures. En fait, la combinaison de tels composés (polymère(s) et tensioactif(s)), alors que leur concentration est très faible, fait apparaître des phases structurées ; lesdites phases présentant un caractère de stabilité les rendant compatibles avec des utilisations dans le domaine cosmétique.

L'invention a donc pour premier objet une composition cosmétique aqueuse comprenant un mélange :
- d'au moins un agent tensioactif anionique, avec une teneur de l'ordre de 0,1 à 0,55% en poids,
- d'au moins un polymère cationique, choisi parmi les hydroxypropyl guars hydroxypropyl trimonium chlorures avec une teneur de l'ordre de 0,01 à 2% en poids, ledit polymère cationique, étant choisi de telle sorte que le ratio de viscosité Vs, défini comme le rapport entre la viscosité maximale en présence de tensioactif et la viscosité en l'absence de tensioactif, soit au minimum supérieur à 2.

Un autre objet de la présente invention est constitué par l'utilisation d'un mélange comprenant :
- de l'ordre de 0,1 à 0,55% en poids d'au moins un agent tensioactif anionique,
- de l'ordre de 0,01 à 2% en poids du polymère cationique choisi parmi les hydroxypropyl guars hydroxypropyl trimonium chlorures, le polymère cationique, étant choisi de telle sorte que le ratio de viscosité Vs, défini comme le rapport entre la viscosité maximale en présence de tensioactif et la viscosité en l'absence de tensioactif, soit au minimum supérieur à 2,
en tant qu'agent structurant de compositions cosmétiques aqueuses.

On a constaté que, de manière particulièrement avantageuse, les compositions cosmétiques aqueuses comprenant le mélange polymère(s) cationique(s) / tensioactif(s) avaient un aspect de gel homogène dont les propriétés étaient au moins équivalentes, et bien souvent meilleures, à celles obtenues avec des polymères associatifs hydrosolubles non ioniques mentionnés auparavant.

En outre, le mélange entrant dans les compositions cosmétiques selon l'invention, a non seulement des propriétés de structuration desdites compositions, mais il apporte de plus des propriétés cosmétiques, ce qui n'était pas ou peu le cas des compositions comprenant les polymères associatifs précités.

Mais d'autres avantages et caractéristiques apparaîtront plus clairement à la lecture de la description, des figures et des exemples qui vont suivre.

Les figures annexées représentent :
Figure 1: l'évolution de la viscosité de compositions cosmétiques aqueuses en fonction de la concentration en poids de tensioactif, pour des compositions conformes à l'invention.
Figure 2 : l'évolution de la viscosité de compositions cosmétiques aqueuses en fonction de la concentration en poids de tensioactif, pour une composition conforme à l'invention et une composition comprenant un polymère associatif selon l'art antérieur.
Figure 3 : l'évolution de la viscosité de compositions cosmétiques aqueuses comprenant un alcool, en fonction de la concentration en poids de tensioactif, pour des compositions conformes à l'invention et des compositions comprenant un polymère associatif selon l'art antérieur.

Il est à noter que dans ce qui va suivre, la nomenclature utilisée pour les composés est conforme à la nomenclature internationale pour les ingrédients cosmétiques (INCI).

Ainsi que cela a été indiqué auparavant, les compositions selon l'invention comprennent un mélange :
- d'au moins un agent tensioactif anionique, avec une teneur de l'ordre de 0,1 à 0,55% en poids,
- d'au moins le polymère cationique choisi parmi les hydroxypropyl guars hydroxypropyl trimonium chlorures, avec une teneur de l'ordre de 0,01 à 2% en poids, ledit polymère cationique, étant choisi de telle sorte que le ratio de viscosité Vs, défini comme le rapport entre la viscosité maximale en présence de tensioactif et la viscosité en l'absence de tensioactif, soit au minimum supérieur à 2.

De façon plus avantageuse, le ratio Vs est compris entre 10 et 50.

La viscosité est mesurée avec un appareil Brookfield, mobile n°4, à 25°C, à une vitesse de 1 tour/minute et à un pH de 6,5.

Selon un mode de réalisation plus particulier de l'invention, les compositions comprennent de l'ordre de 0,1 à 1% en poids, d'au moins un agent tensioactif anionique. Selon un mode de réalisation préféré de l'invention, la teneur en tensioactif anionique est comprise entre 0,35 et 0,55 % en poids.

En ce qui concerne la teneur en polymère cationique, celle-ci est plus particulièrement comprise entre 0,01 à 1 % en poids de polymère cationique, de préférence de l'ordre de 0,01 à 0,5 % en poids de polymère cationique. De manière encore plus préférée, la teneur en polymère cationique est comprise entre 0,05 et 0,5 % en poids.

Les polymères cationiques mis en oeuvre dans le cadre de la présente invention présentent plus particulièrement un caractère associatif fort en présence de tensioactifs anioniques.

Ce caractère associatif se traduit le plus souvent par un phénomène de séparation de phase d'un complexe polymère / tensioactif lors de la dilution d'une solution mère.

Ce caractère associatif peut notamment être identifié par l'emploi d'un test simple consistant à préparer une solution de polymère cationique à tester (0,3% poids) dans un mélange de sodium laureth-2 sulfate (9% poids) / disodium cocoamphodiacétate (3%). Cette solution, dont le pH est ajusté à 6, est ensuite diluée de façon incrémentale en ajoutant de l'eau désionisée et la transmittance à 600 nm mesurée. Si lors de la dilution, la transmittance devient inférieure à 90%, cela traduit la présence d'un phénomène de séparation de phases caractérisant le comportement associatif du polymère à tester.

Notons que tous les polymères cationiques n'ont pas un tel comportement, comme par exemple, les polymères cationiques comme les Polyquaternium-7 ou Polyquaternium-2.

Parmi les dérivés de guar, on cite l'hydroxypropyl guar hydroxypropyl trimonium chlorure (JAGUAR C162 commercialisés par RHODIA).

Les polymères cationiques présentent plus particulièrement une masse molaire en poids d'au moins 2000 g/mol et plus préférentiellement comprise entre 2.10⁴ et 3.10⁶ g/mol, selon leur degré de polymérisation éventuelle. Les masses molaires en poids des polymères sont habituellement mesurées par exclusion de taille. Eventuellement, elles peuvent être mesurées directement par diffusion de la lumière ou à partir de la viscosité intrinsèque en utilisant un étalonnage selon : "Viscosity-Molecular weight relationship, intrinsic chain flexibility and dynamic solution properties of guar galactomannan" de G. Robinson, S.B. Ross Murphy, E.R. Morris, Carbohydrate Research 107, p.17-32, 1982.

Le degré d'hydroxyalkylation (substitution molaire ou MS) est de préférence compris entre 0 et 1,2. Toujours dans le cas de ces polymères, le degré de cationicité (degré de substitution ou DS) est plus particulièrement compris entre 0,01 et 0,6. C'est par exemple le cas des Jaguars C162 et C2000 commercialisés par la société Rhodia Chimie.

Le polymère cationique est associé à au moins un tensioactif anionique.

Parmi les agents tensioactifs anioniques susceptibles d'être utilisés dans le cadre de cette invention, on peut citer à titre d'exemples :
- les alkylesters sulfonates de formule R-CH(SO₃M)-COOR', où R représente un radical alkyle en C₈-C₂₀, de préférence en C₁₀-C₁₆, R' un radical alkyle en C₁-C₆, de préférence en C₁-C₃, et M est un cation alcalin (sodium, potassium, lithium), un ion ammonium, substitué ou non (méthyl-, diméthyl-, triméthyl-, tetraméthylammonium, diméthylpiperidinium ...) ou dérivé d'une alcanolamine (monoéthanolamine, diéthanolamine, triéthanolamine ...).

On peut citer tout particulièrement les méthyl ester sulfonates dont le radical R est en C₁₄-C₁₆;
- les alkylsulfates de formule ROSO₃M, où R représente un radical alkyle ou hydroxyalkyle en C₁₀-C₂₄, de préférence en C₁₂-C₂₀ et tout particulièrement en C₁₂-C₁₈, M représentant un atome d'hydrogène ou un cation de même définition que ci-dessus, ainsi que leurs dérivés oxyalkylénés (oxyéthylénés et/ou oxypropylénés), présentant en moyenne de 0,5 à 6 motifs, de préférence de 0,5 à 3 motifs oxyalkylénés ;
- les alkylamides sulfates de formule RCONHR'OSO₃M où R représente un radical alkyle en C₂-C₂₂, de préférence en C₆-C₂₀, R' un radical alkyle en C₂-C₃, M un atome d'hydrogène ou un cation de même définition que ci-dessus, ainsi que leurs dérivés oxyalkylénés (oxyéthylénés et/ou oxypropylénés), présentant en moyenne de 0,5 à 60 motifs oxyalkylénés ;
- les sels d'acides gras saturés ou insaturés en C₈-C₂₄, de préférence en C₁₄-C₂₀, les alkylbenzènesulfonates en C₉-C₂₀, les alkylsulfonates primaires ou secondaires en C₈-C₂₂, les alkylglycérol sulfonates, les acides polycarboxyliques sulfonés décrits dans GB-A-1 082 179, les sulfonates de paraffine, les N-acyl N-alkyltaurates, les alkylphosphates, les alkyliséthionates, les alkylsuccinamates les alkylsulfosuccinates, les monoesters ou diesters de sulfosuccinates, les N-acyl sarcosinates, les sulfates d'alkylglycosides, les polyéthoxycarboxylates.

Selon un mode de réalisation préféré de l'invention, on met en oeuvre en tant que tensioactif anionique, les dérivés d'alcools gras ou d'acides gras sulfatés oxyalkylénés, et de préférence les dérivés oxyéthylénés. Les dérivés de l'alcool laurique comme le lauryl éther sulfate de sodium, de magnésium, ou leurs mélanges, conviennent particulièrement bien à la réalisation de l'invention.

On ne sortirait pas du cadre de la présente en utilisant un ou plusieurs tensioactifs anioniques. Ainsi, parmi les tensioactifs anioniques de préférence mis en oeuvre avec des tensioactifs anioniques de type sulfates, on peut citer les sulfosuccinates, les mono-alkyl phosphates, ou les alkyl éther carboxylates.

Le tensioactif anionique peut être éventuellement associé à au moins un co-tensioactif amphotère ou zwitterionique.

Parmi les agents tensioactifs susceptibles d'entrer comme constituant de la composition selon l'invention, on peut citer les dérivés d'aminoacides comme les bétaïnes, qui sont des composés zwitterioniques, ou les amphotères.

Par agent tensioactif zwitterionique de type bétaïne, on entend un agent tensioactif portant sur la même molécule une charge négative et une charge positive permanente en fonction du pH et ne présentant pas de point isoélectrique. Il s'agit de dérivés quaternisés et à titre d'exemples, on peut citer :
- les bétaïnes : 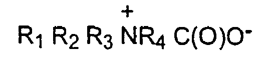 comme la lauryl bétaine (Mirataine BB de la société Rhodia Chimie)
- les sulfo-bétaïnes : 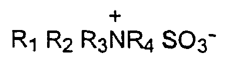
- les amidoalkylbétaïnes : 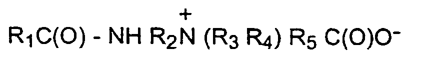 comme la cocamidopropyl bétaine (Mirataine BDJ de la société Rhodia Chimie)
- les sulfo-bétaïnes : 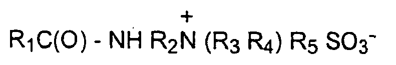 comme la cocamidopropyl hydroxy sultaine (Mirataine CBS de la société Rhodia Chimie).

Dans les formules précitées, les radicaux R₁ représentent un radical alkyle ou alcényle de 10 à 24 atomes de carbone, R₂, R₃, R₄, et R₅ identiques ou différents représentent un radical alkyle ou alcényle ayant de 1 à 4 atomes de carbone.

Par agent tensioactif amphotère, on entend un agent tensioactif portant simultanément une charge anionique et une charge cationique, produit dont le degré d'ionisation variera en fonction du pH du milieu dans lequel il se trouve. Ces produits présentent un point isoélectrique (IEP) compris entre 3,5 et 6,5. Ce point isoélectrique pourra être ajusté par le contrôle des sous-produits du produit.

A titre d'exemple, on peut citer :
- les cocoamphoacétates et cocoamphodiacétates répondant plus généralement à la formule : 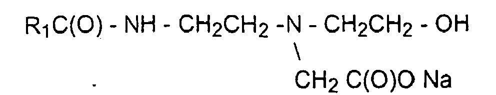 formule dans laquelle R₁ représente un radical alkyle ou alcényle de 10 à 24 atomes de carbone, et représente plus particulièrement des chaînes coco et lauryle. (Miranol C2M et Miranol Ultra C32 de la société Rhodia Chimie) ;
- les alkylampho-propionates ou -dipropionates, (Miranol C2M SF de la société Rhodia Chimie)
- les alkyl amphohydroxypropyl sultaines (Miranol CS de la société Rhodia Chimie)

On peut également utiliser des tensioactifs de type non ionique comme les dérivés éthoxylés d'esters de sorbitan, les alkylpolyglucosides, les alkylglucosamides, ou les acides, alcools, amides, ou amines grasses éthoxylés.

Une variante particulièrement avantageuse de la présente invention consiste à mettre en oeuvre au moins un tensioactif choisi parmi les sulfates, éventuellement combiné à un co-tensioactif, ionique ou non.

Dans le cadre de cette variante, la concentration en tensioactif et éventuellement co-tensioactif, est plus avantageusement comprise 10⁻² et 10 fois la valeur de la concentration micellaire critique du tensioactif ou du mélange de tensioactifs. De préférence, cette concentration est comprise entre 0,5 et 1,5 fois la valeur de la concentration micellaire critique.

Ces mélanges polymère(s) cationique(s) / tensioactif(s) peuvent être préparés de façon courante. Par exemple, on peut préparer une solution/suspension de polymère dans l'eau selon des méthodes connues de l'homme de l'art, suivie de l'addition d'un ou plusieurs tensioactifs jusqu'à obtention de la rhéologie souhaitée.

Les mélanges qui viennent d'être décrits peuvent être avantageusement utilisés dans des compositions cosmétiques aqueuses, en tant qu'agent texturant.

En effet, le mélange selon l'invention permet d'augmenter de manière très importante la viscosité de la composition cosmétique dans laquelle il est introduit, jusqu'à gélifier cette composition.

Ce mélange est ensuite utilisé pour l'obtention de compositions aqueuses cosmétiques.

On entend par le terme compositions (ou formulations) cosmétiques, tous les produits ou préparations cosmétiques comme celles décrites dans l'annexe I ("Illustrative list by category of cosmetic products") de la directive européenne n° 76/768/CEE du 27 juillet 1976, dite directive cosmétique.

Les compositions cosmétiques faisant l'objet de l'invention peuvent être formulées en un grand nombre de types de produits pour la peau et/ ou le cheveu, les gels (coiffants notamment), les conditionneurs, les formulations pour le coiffage ou pour faciliter le peignage des cheveux, les lotions pour les mains et le corps, les produits régulant l'hydratation de la peau, les laits de toilette, et bien d'autres compositions du même type.

Les compositions cosmétiques faisant l'objet de l'invention peuvent ainsi, par exemple, contenir des dérivés de silicones, solubles ou non dans l'eau de la composition cosmétique.

Parmi les dérivés de silicones solubles dans l'eau de la composition, on peut citer entre autres, les diméthicones copolyols (Mirasil DMCO commercialisée par la société Rhodia Chimie).

Pour ce qui a trait aux silicones se présentant sous forme de dispersions insolubles dans l'eau de la composition, on peut utiliser de manière convenable, des organopolysiloxanes non hydrosolubles et non volatils (appelés également par la suite "silicones non hydrosolubles et non volatils") parmi lesquels on peut citer les huiles, gommes ou résines polyalkylsiloxanes, polyarylsiloxanes, polyalkylarylsiloxanes, ou leurs dérivés fonctionnalisés non hydrosolubles, ou leurs mélanges, non volatils.

Lesdits organopolysiloxanes sont considérés comme non hydrosolubles et non volatils, lorsque leur solubilité dans l'eau est inférieure à 50 g/litre et leur viscosité intrinsèque d'au moins 3000 mPa.s, à 25°C.

A titre d'exemples d'organopolysiloxanes ou silicones non hydrosolubles et non volatils, on peut citer des gommes silicones comme par exemple la gomme diphényl diméthicone commercialisée par la société Rhodia Chimie, et de préférence les polydiméthylsiloxanes présentant une viscosité au moins égale à 6.10⁵ mPa.s, à 25°C, et de façon encore plus préférentielle, ceux d'une viscosité supérieure à 2. 10⁶ mPa.s, à 25°C, tels que la Mirasil DM 500000^{®} commercialisée par la société Rhodia Chimie.

Selon l'invention, l'organopolysiloxane ou silicone non hydrosoluble et non volatil se trouve sous une forme dispersée au sein de la composition cosmétique le renfermant.

Celui-ci se présente sous forme de particules dont la taille peut être choisie en fonction de la nature de la composition cosmétique ou de la performance recherchée pour ladite composition. D'une manière générale, cette taille peut varier de 0,01 à 70 microns.

D'une manière préférentielle, cette taille est de l'ordre de 0,1 à 50 microns, tout particulièrement de l'ordre de 1 à 30 microns.

Pour faciliter leur mise en oeuvre, ces organopolysiloxanes peuvent être préalablement dispersés ou solubilisés dans des dérivés silicones de faible viscosité, volatils ou non, puis émulsionnés dans la composition cosmétique.

Parmi ces silicones de faible viscosité, on peut citer les silicones volatils cycliques et les polydiméthyl siloxanes de faible masse.

On pourra également utiliser des dérivés de silicones fonctionnalisés comme les dérivés aminés directement sous forme d'émulsions ou à partir d'une micro-émulsion préformée comme la Mirasil ADM-E (Amodiméthicone) commercialisée par la société Rhodia Chimie.

Il est de même envisageable d'utiliser des huiles pouvant exercer des fonctions conditionnantes, protectrices, ou émollientes. De telles huiles sont généralement choisies parmi les alkylmonoglycérides, les alkyldiglycérides, les triglycérides comme les huiles extraites des plantes et des végétaux (huiles de palme, de coprah, de graine de coton, de soja, de tournesol, d'olive, de pépin de raisin, de sésame, d'arachide, de ricin...) ou les huiles d'origine animale (suif, huiles de poisson...), des dérivés de ces huiles comme les huiles hydrogénées, les dérivés de la lanoline, le petrolatum, les huiles minérales ou les huiles paraffiniques, le perhydrosqualane, le squalène, les diols comme le 1-2-dodécanediol, l'alcool cétylique, l'alcool stéarylique, l'alcool oléique, les esters gras comme le palmitate d'isopropyl, le cocoate d'éthyl-2-hexyl, le myristyl myristate, les esters de l'acide lactique, l'acide stéarique, l'acide béhénique, l'acide isostéarique.

On peut aussi incorporer dans la composition cosmétique, sous forme de dispersions ou de solutions, des agents bactéricides ou fongicides afin d'améliorer la désinfection de la peau, comme par exemple le triclosan ; des agents anti-pelliculaires, comme notamment le zinc pyrithione ou l'octopyrox ; des agents insecticides comme les pyréthroides naturels ou de synthèse.

Les compositions cosmétiques peuvent également contenir des agents pour la protection de la peau et/ou des cheveux contre les agressions du soleil et des rayons UV. Ainsi, les compositions peuvent comprendre des filtres solaires qui sont des composés chimiques absorbant fortement le rayonnement UV, comme les composés autorisés dans la directive européenne N° 76/768/CEE, ses annexes et les modifications ultérieures de cette directive.

Dans le cas où les divers éléments constitutifs de la composition cosmétique présentent une solubilité trop faible dans la composition ou lorsqu'ils se présentent sous forme solide à température ambiante, lesdits éléments constitutifs peuvent avantageusement être solubilisés dans un véhicule organique, comme des huiles minérales ou naturelles, des dérivés de silicones, des cires, ou bien encore encapsulés dans des matrices comme des polymères de type latex.

Les compositions cosmétiques faisant l'objet de l'invention peuvent également contenir des résines fixatives.

Ces résines fixatives, lorsqu'elles sont présentes, le sont généralement à des concentrations comprises entre 0,01 et 10%, préférentiellement entre 0,5 et 5%.

Les résines fixatives entrant dans les compositions cosmétiques sont plus particulièrement choisies parmi les résines suivantes :
- les copolymères acrylate de méthyle / acrylamide, copolymères polyvinylméthyléther / anhydride maléique, copolymères acétate de vinyle / acide crotonique, copolymères octylacrylamide / acrylate de méthyle / butylaminoéthylméthacrylate, polyvinylpyrrolidones, copolymères polyvinylpyrrolidone / méthacrylate de méthyle, copolymères polyvinylpyrrolidone / acétate de vinyle, alcools polyvinyliques, copolymères alcool polyvinylique / acide crotonique, copolymères alcool polyvinylique / anhydride maléique, hydroxypropyl celluloses, hydroxypropyl guars, polystyrène sulfonates de sodium, terpolymères polyvinylpyrrolidone / éthyl méthacrylate/ acide méthacrylique, monométhyl éthers de poly(méthylvinyl éther / acide maléique), polyvinylacétates greffés sur des troncs polyoxyéthylènes (EP-A-219 048),
- les copolyesters dérivés d'acide, anhydride ou d'un diester téréphtalique et/ou isophtalique et/ou sulfoisophtalique et d'un diol, tels que :
   - les copolymères polyesters à base de motifs ethylène téréphtalate et/ou propylène téréphtalate et polyoxyéthylène téréphtalate, (US-A-3 959 230, US-A-3 893 929, US-A-4 116 896, US-A-4 702 857, US-A-4 770 666) ;
   - les oligomères polyesters sulfonés obtenus par sulfonation d'un oligomère dérivé de l'alcool allylique éthoxylé, du diméthyltéréphtalate et du 1,2 propylène diol (US-A-4 968 451) ;
   - les copolymères polyesters dérivés de diméthyltéréphtalate, d'acide isophtalique, de sulfoisophtalate de diméthyl et d'éthylène glycol (EP-A-540374)
   - les copolymères comprenant des unités polyesters dérivés de diméthyltéréphtalate, d'acide isophtalique, de sulfoisophtalate de diméthyl et d'éthylène glycol et d'unités polyorganosiloxanes (FR-A-2 728 915).
   - les oligomères polyesters sulfonés obtenus par condensation de l'acide isophtalique, du sulfosuccinate de diméthyle et de diéthylène glycol (FR-A-2 236 926)
   - les copolymères polyesters à base de motifs propylène téréphtalate et polyoxyéthylène téréphtalate et terminés par des motifs éthyles, méthyles (US-A-4 711 730) ou des oligomères polyesters terminés par des groupes alkylpolyéthoxy (US-A-4 702 857) ou des groupes anioniques sulfopolyéthoxy (US-A-4 721 580), sulfoaroyles (US-A-4 877 896) ;
   - les polyesters-polyuréthanes obtenus par réaction d'un polyester obtenu à partir d'acide adipique et/ou d'acide téréphtalique et/ou d'acide sulfoisophtalique et d'un diol, sur un prépolymère à groupements isocyanates terminaux obtenus à partir d'un polyoxyéthylène glycol et d'un diisocyanate (FR-A-2 334 698) ;
- les monoamines ou polyamines éthoxylées, les polymères d'amines éthoxylées (US-A-4 597 898, EP-A-11 984).

De manière préférentielle, les résines fixatives sont choisies parmi les polyvinylpyrrolidone (PVP), copolymères de polyvinylpyrrolidone et de méthyl méthacrylate, copolymère de polyvinylpyrrolidone et d'acétate de vinyle (VA), copolymères polytéréphtale d'éthylène glycol / polyéthylène glycol, copolymères polytéréphtalate d'éthylène glycol / polyéthylène glycol / polyisophtalate sulfonate de sodium, et leurs mélanges.

Ces résines fixatives sont préférentiellement dispersées ou solubilisées dans le véhicule choisi.

Les compositions cosmétiques faisant l'objet de l'invention peuvent également contenir des dérivés polymériques exerçant une fonction protectrice.

Ces dérivés polymériques peuvent être présents en quantités de l'ordre de 0,01-10%, de préférence environ 0,1-5%, et tout particulièrement de l'ordre de 0,2-3% en poids.

Ces agents peuvent notamment être choisis parmi :
- les dérivés cellulosiques non ioniques tels que les hydroxyéthers de cellulose, la méthylcellulose, l'éthylcellulose, l'hydroxypropyl méthylcellulose, l'hydroxybutyl méthylcellulose ;
- les polyvinylesters greffés sur des troncs polyalkylénés tels que les polyvinylacétates greffés sur des troncs polyoxyéthylènes (EP-A-219 048);
- les alcools polyvinyliques.

Les compositions cosmétiques faisant l'objet de l'invention peuvent aussi comprendre des agents plastifiants.

Lesdits agents, s'ils sont présents, peuvent représenter entre 0,1 à 20% de la formulation de préférence de 1 à 15%.

Parmi les agents plastifiants particulièrement utiles, on peut citer les adipates, les phtalates, les isophtalates, les azélates, les stéarates, les silicones copolyols, les glycols, l'huile de ricin, ou leurs mélanges.

On peut aussi avantageusement ajouter à ces compositions des agents séquestrants des métaux, plus particulièrement ceux séquestrant le calcium comme les ions citrates.

On peut également incorporer aux compositions cosmétiques faisant l'objet de l'invention des agents humectants, parmi lesquels figurent, entre autres, le glycérol, le sorbitol, l'urée, le collagène, la gélatine, l'aloe vera, l'acide hyaluronique ou des solvants volatils hydrosolubles comme l'éthanol ou le propylène glycol dont les teneurs peuvent atteindre jusqu'à 60% en poids de la composition.

Pour diminuer encore l'irritation ou l'agression du cuir chevelu, on peut aussi ajouter des polymères hydrosolubles ou hydrodispersables comme le collagène ou certains dérivés non allergisants de protéines animales ou végétales (hydrolysats de protéines de blé par exemple), des hydrocolloïdes naturels (gomme de guar, de caroube, de tara, ...) ou issus de procédés de fermentation et les dérivés de ces polycarbohydrates comme les celluloses modifiées non ioniques comme par exemple l'hydroxyéthylcellulose, ou anionique comme la carboxyméthylcellulose ; les dérivés du guar ou de la caroube comme leurs dérivés non-ioniques (par exemple hydroxypropylguar) ou les dérivés anioniques (carboxyméthylguar et carboxyméthylhydroxypropylguar).

A ces composés, on peut ajouter en association, des poudres ou des particules minérales comme du carbonate de calcium, du bicarbonate de sodium, du dihydrogénophosphate de calcium, des oxydes minéraux sous forme de poudre ou sous forme colloïdale (particules de taille inférieure ou de l'ordre de un micromètre, parfois de quelque dizaines de nanomètres) comme du dioxyde de titane, de la silice, des sels d'aluminium utilisés généralement comme anti-transpirants, du kaolin, du talc, des argiles et leurs dérivés, etc..

Des agents conservateurs comme les méthyl, éthyl, propyl et butyl esters de l'acide p-hydroxybenzoïque, le benzoate de sodium, le GERMABEN^{®} ou tout agent chimique évitant la prolifération des bactéries ou des moisissures et utilisé traditionnellement des les compositions cosmétiques, peuvent aussi être introduits dans les compositions aqueuses cosmétiques selon l'invention, généralement à hauteur de 0,01 à 3 % en poids.

La quantité de ces produits est habituellement ajustée pour éviter toute prolifération de bactéries, moisissures ou levures dans les compositions cosmétiques.

Alternativement à ces agents chimiques, on peut parfois utiliser des agents modifiant l'activité de l'eau et augmentant fortement la pression osmotique comme les carbohydrates ou des sels.

Pour protéger la peau et/ou les cheveux des agressions du soleil et des rayons UV, on peut ajouter à ces formulations des particules minérales comme l'oxyde de zinc, le dioxyde de titane ou les oxydes de cérium sous forme de poudre ou de particules colloïdales, seuls ou en mélange. Ces poudres peuvent éventuellement être traitées en surface pour augmenter l'efficacité de leur action anti-UV ou pour faciliter leur incorporation dans les formulations cosmétiques ou pour inhiber la photoréactivité de surface.

A ces ingrédients on peut ajouter, si nécessaire, et dans le but d'augmenter le confort lors de l'utilisation de la composition par le consommateur, un ou des parfums, des agents colorants parmi lesquels on peut citer les produits décrits dans l'annexe IV ("List of colouring agents allowed for use in cosmetic products") de la directive européenne n° 76/768/CEE du 27 juillet 1976 dite directive cosmétique, et/ou des agents opacifiants comme des pigments.

Bien que cela ne soit pas obligatoire, la composition peut aussi contenir des polymères viscosants ou gélifiants de façon à ajuster la texture de la composition, comme les polyacrylates réticulés (Carbopol commercialisés par Goodrich), les dérivés non cationiques de la cellulose comme l'hydroxypropylcellulose, la carboxyméthylcellulose, les guars et leurs dérivés non ioniques, la gomme xanthane et ses dérivés, utilisés seuls ou en association, ou les mêmes composés, généralement sous la forme de polymères hydrosolubles modifiés par des groupements hydrophobes liés de manière covalente au squelette polymère comme décrit dans le brevet WO 92/16187 et/ou de l'eau pour amener le total des constituants de la formulation à 100 %.

Les compositions cosmétiques faisant l'objet de l'invention peuvent également contenir des agents dispersants polymériques en quantité de l'ordre de 0,1-7% en poids, pour contrôler la dureté en calcium et magnésium, agents tels que:
les sels hydrosolubles d'acides polycarboxyliques de masse moléculaire en poids de l'ordre de 2000 à 100000 g/mol, obtenus par polymérisation ou copolymérisation d'acides carboxyliques éthyléniquement insaturés tels que l'acide acrylique, l'acide ou l'anhydride maléique, l'acide fumarique, l'acide itaconique, l'acide aconitique, l'acide mésaconique, l'acide citraconique, l'acide méthylènemalonique, et tout particulièrement les polyacrylates de masse moléculaire en poids de l'ordre de 2 000 à 10 000 g/mol (US-A-3 308 067), les copolymères d'acide acrylique et d'anhydride maléique de masse moléculaire en poids de l'ordre de 5000 à 75 000 g/mol (EP-A-66 915) ;
. les polyéthylèneglycols de masse moléculaire en poids de l'ordre de 1000 à 50000 g/mol.

Les exemples suivants sont donnés à titre illustratif et ne peuvent limiter la portée de l'invention.

### EXEMPLE 1

On prépare une solution à 1 % poids, dans l'eau distillée, des polymères cationiques listés dans le tableau ci-dessous, en mettant en oeuvre les méthodes classiques.

Le pH est ajusté à 6,5.

| | **Nom INCl** | **Origine** | **Nom commercial** | **Fournisseur** |
|---|---|---|---|---|
| **Produit A** | Hydroxypropyl guar hydroxypropyl trimonium chloride | Guar | Jaguar C162 | Rhodia |
| **Produit B** | guar hydroxypropyl trimonium chloride | Guar | Jaguar C13S | Rhodia |
| (comparatif) | | | | |
| **Produit C** | Polyquaternium-10 | Cellulose | Polymer JR400 | Union Carbide |
| (comparatif) | | | | |
| **Produit D** | guar hydroxypropyl trimonium chloride | Guar | Jaguar Excel | Rhodia |
| (comparatif) | | | | |

A ces solutions sont ajoutées des concentrations croissantes de sodium laureth-2 sulfate (Empicol ESB3M, 28% actif, employé tel que).

Après une heure de repos à température ambiante, la viscosité (Brookfield, hélice 4, 1 tour/minute) est mesurée.

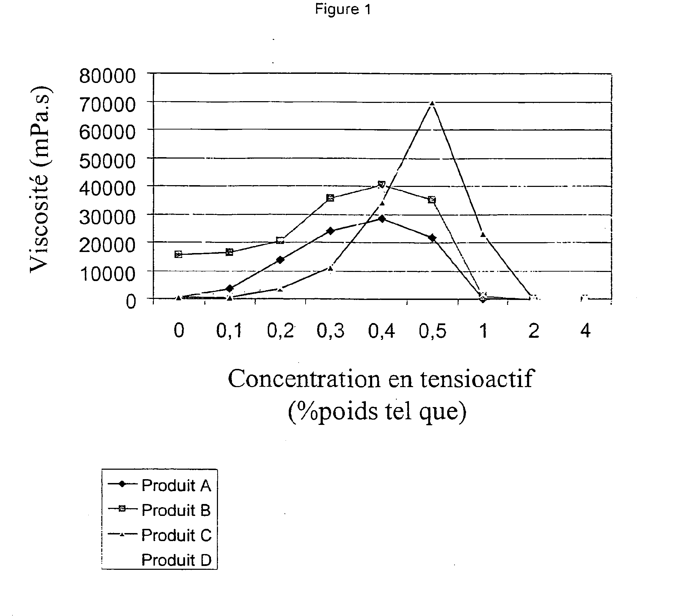

Les résultats sont repris dans le graphe de la figure 1.

On constate que les ratios de viscosité Vs, définis comme le rapport entre viscosité maximale en présence de tensioactif et viscosité en l'absence de tensioactif varient entre 2,6 et 57 en fonction de la nature du polymère cationique.

### EXEMPLE 2

Les propriétés du polymère cationique D sont comparées avec celles d'un épaississant associatif classique, le tétradécyl hydroxypropyl guar préparé par condensation des chaînes en C14 sur un hydroxypropyl guar (voir EP 281360).

On prépare une solution des polymères décrits dans le tableau ci-dessous, à 1 % poids dans l'eau distillée, en mettant en oeuvre les méthodes classiques.

Le pH est ajusté à 6,5.

| | **Nom INCl** | **Origine** | **Nom commercial** | **Fournisseur** |
|---|---|---|---|---|
| **Produit E** | Tetradecyl hydroxypropyl guar | Guar | - | Rhodia |
| (comparatif) | | | | |
| **Produit D** | guar hydroxypropyl trimonium chloride | Guar | Jaguar Excel | Rhodia |
| (comparatif) | | | | |

A ces solutions sont ajoutées des concentrations croissantes de sodium laureth-2 sulfate (Empicol ESB3M, 28% actif, employé tel que).

Après une heure de repos à température ambiante, la viscosité (Brookfield, hélice 4, 1 tour/minute) est mesurée.

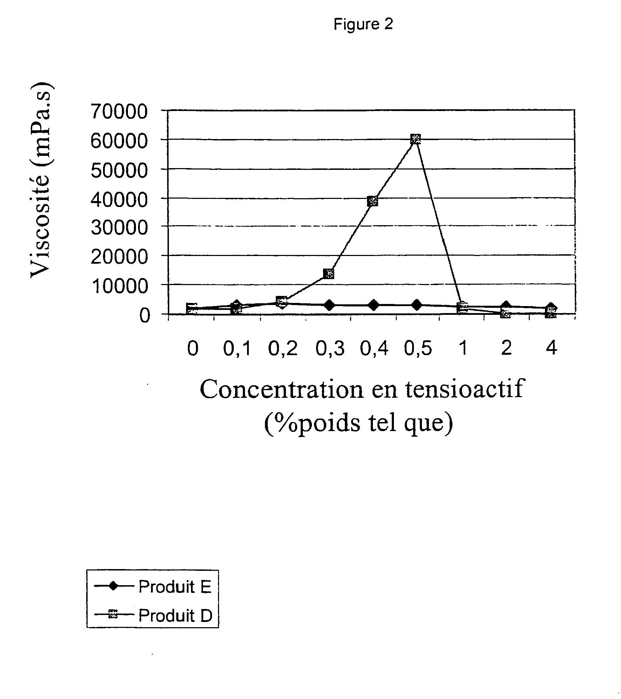

Les résultats sont repris dans le graphe de la figure 2.

Les ratios de viscosité Vs, définis comme le rapport entre viscosité maximale en présence de tensioactif et viscosité en l'absence de tensioactif sont de 1,45 pour la technologie traditionnelle contre 33,3 pour le polymère cationique.

### EXEMPLE 3

La compatibilité des polymères cationiques en présence d'alcool a été testée.

On prépare une solution des polymères cationiques (1% poids) décrits dans le tableau ci-dessous, dans l'eau distillée contenant des proportions variables d'éthanol (20%, 40%, 60% pour le produit A ; 20% et 60% pour le produit C), en mettant en oeuvre les méthodes classiques.

Le pH est ajusté à 6,5.

| | **Nom INCl** | **Origine** | **Nom commercial** | **Fournisseur** |
|---|---|---|---|---|
| **Produit A** | Hydroxypropyl guar hydroxypropyl trimonium chloride | Guar | Jaguar C162 | Rhodia |
| **Produit C** | Polyquaternium-10 | Cellulose | Polymer JR400 | Union Carbide |
| (comparatif) | | | | |

A ces solutions sont ajoutées des concentrations croissantes de sodium laureth-2 sulfate (Empicol ESB3M, 28% actif, employé tel que).

Après une heure de repos à température ambiante, la viscosité (Brookfield, hélice 4, 1 tour/minute) est mesurée.

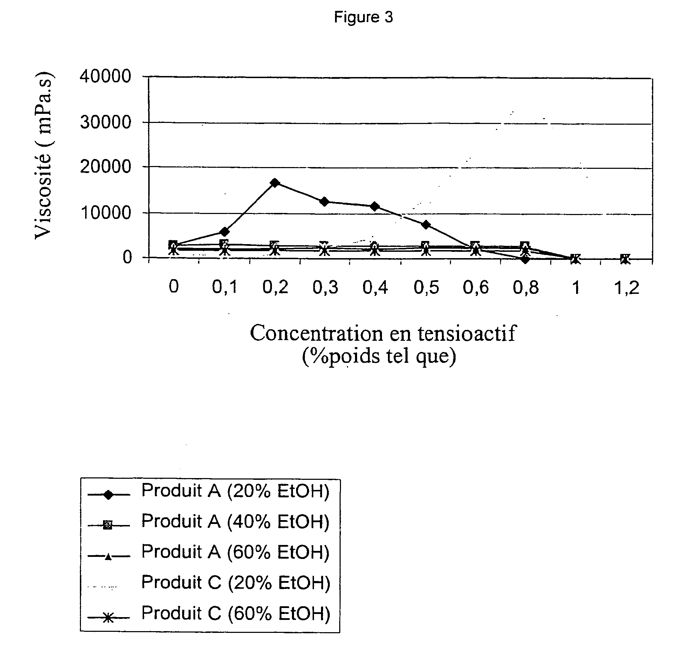

Les résultats sont repris dans le graphe de la figure 3.

Les deux polymères cationiques montrent une bonne compatibilité en milieu hydroalcoolique avec des ratios de viscosité supérieurs à 2 pour des teneurs en alcool inférieures à 40%. La position du pic de viscosité maximale dépend de la nature du polymère cationique.

## Revendications

1. Compositions aqueuses cosmétiques comprenant un mélange :
- d'au moins un agent tensioactif anionique, avec une teneur de l'ordre de 0,1 à 0,55% en poids,
- d'au moins un polymère cationique choisi parmi les hydroxypropyl guars hydroxypropyl trimonium chlorures, avec une teneur de l'ordre de 0,01 à 2% en poids, ledit polymère cationique étant choisi de telle sorte que le ratio de viscosité Vs, défini comme le rapport entre la viscosité maximale en présence de tensioactif et la viscosité en l'absence de tensioactif, soit au minimum supérieur à 2.

2. Composition selon l'une des revendications précédentes, **caractérisée en ce qu'**elle comprend de l'ordre de 0,05 à 0,5% en poids de polymère cationique.

3. Composition selon l'une des revendications précédentes, **caractérisée en ce que** le tensioactif anionique est choisi parmi :
. les alkylesters sulfonates de formule R-CH(SO₃M)-COOR', où R représente un radical alkyle en C₈-C₂₀, R' un radical alkyle en C₁-C₆, et M un cation alcalin, ammonium substitué ou non ou dérivé d'une alcanolamine ;
. les alkylsulfates de formule ROSO₃M, où R représente un radical alkyle ou hydroxyalkyle en C₁₀-C₂₄, M représentant un atome d'hydrogène ou un cation alcalin, ammonium substitué ou non ou dérivé d'une alcanolamine, ainsi que leurs dérivés oxyalkylénés présentant en moyenne de 0,5 à 6 motifs oxyalkylénés ;
. les alkylamides sulfates de formule RCONHR'OSO₃M où R représente un radical alkyle en C₂-C₂₂, R' un radical alkyle en C₂-C₃, M représentant un atome d'hydrogène ou un cation alcalin, ammonium substitué ou non ou dérivé d'une alcanolamine, ainsi que leurs dérivés oxyalkylénés présentant en moyenne de 0,5 à 60 motifs oxyalkylénés ;
. les sels d'acides gras saturés ou insaturés en C₈-C₂₄ ;
. les alkylbenzènesulfonates en C₉-C₂₀, les alkylsulfonates primaires ou secondaires en C₈-C₂₂, les alkylglycérol sulfonates, les acides polycarboxyliques sulfonés, les sulfonates de paraffiné, les N-acyl N-alkyltaurates, les alkylphosphates, les alkyliséthionates, les alkylsuccinamates les alkylsulfosuccinates, les monoesters ou diesters de sulfosuccinates, les N-acyl sarcosinates, les sulfates d'alkylglycosides, les polyéthoxycarboxylates.

4. Composition selon l'une quelconque des revendications précédentes, **caractérisée en ce qu'**elle comprend au moins un co-tensioactif.

5. Utilisation d'un mélange comprenant :
- de l'ordre de 0,1 à 0.55% en poids d'au moins un agent tensioactif anionique,
- de l'ordre de 0,01 à 2% en poids d'au moins un polymère cationique choisi parmi les hydroxypropyl quars hydroxypropyl trimonium chlorures, le polymère cationique étant choisi de telle sorte que le ratio de viscosité Vs, défini comme le rapport entre la viscosité maximale en présence de tensioactif et la viscosité en l'absence de tensioactif, soit au minimum supérieur à 2,
en tant qu'agent structurant de compositions cosmétiques aqueuses.

## Claims

1. Aqueous cosmetic compositions comprising a mixture:
• of at least one anionic surfactant, in an amount of the order of 0.1% to 0.55% by weight;
• of at least one cationic polymer selected from hydroxypropyl guars hydroxypropyl trimonium chlorides, in an amount of the order of 0.01% to 2% by weight, said cationic polymer being selected so that the viscosity ratio Vs, defined as the ratio between the maximum viscosity in the presence of surfactant and the viscosity in the absence of surfactant, is a minimum of 2.

2. A composition according to any one of the preceding claims, **characterized in that** it comprises of the order of 0.05% to 0.5% by weight of cationic polymer.

3. A composition according to any one of the preceding claims, **characterized in that** the anionic surfactant is selected from:
• alkylester sulphonates with formula R-CH(SO₃M)-COOR', where R represents a C₈-C₂₀ alkyl radical, R' represents a C₁-C₆ alkyl radical, and M represents an alkali cation or substituted or non substituted or alkanolamine-derived ammonium;
• alkylsulphates with formula ROSO₃M, where R represents a C₁₀-C₂₄ alkyl or hydroxyalkyl radical, M represents a hydrogen atom or an alkali cation or substituted or non substituted or alkanolamine-derived ammonium, and their oxyalkylenated derivatives with an average of 0.5 to 6 oxyalkylenated motifs;
• alkylamide sulphates with formula RCONHR'OSO₃M where R represents a C₂-C₂₂ alkyl radical, R' represents a C₂-C₃ alkyl radical, M represents a hydrogen atom or an alkali cation, or a substituted or non substituted or alkanolamine-derived ammonium, and their oxyalkylenated derivatives with an average of 0.5 to 60 motifs oxyalkylenated motifs;
• salts of saturated or unsaturated C₈-C₂₄ fatty acids;
• C₉-C₂₀ alkylbenzenesulphonates, primary or secondary C₈-C₂₂ alkylsulphonates, alkylglycerolsulphonates, sulphonated polycarboxylic acids, paraffin sulphonates, N-acyl-N-alkyltaurates, alkylphosphates, alkylisethionates, alkylsuccinamates, alkylsulphosuccinates, sulphosuccinate monoesters or diesters, N-acylsarcosinates, alkylglycoside sulphates or polyethoxycarboxylates.

4. A composition according to any one of the preceding claims, **characterized in that** it comprises at least one co-surfactant.

5. Use of a mixture comprising:
• of the order of 0.1% to 0.55% by weight of at least one anionic surfactant;
• of the order of 0.01% to 2% by weight of at least one cationic polymer selected from hydroxypropyl guars hydroxypropyl trimonium chlorides, the cationic polymer being selected so that the viscosity ratio Vs, defined as the ratio between the maximum viscosity in the presence of surfactant and the viscosity in the absence of surfactant, is a minimum of 2;
as a structuring agent for aqueous cosmetic compositions.

## Patentansprüche

1. Wässrige kosmetische Zusammensetzung, welche ein Gemisch aufweist aus:
- wenigstens einem anionischen Tensid, mit einem Gehalt im Bereich von 0,1 bis 0,55 Gew.-%,
- wenigstens einem kationischen Polymer, gewählt aus den Hydroxypropylguarhydroxypropyltrimoniumchloriden, mit einem Gehalt im Bereich von 0,01 bis 2 Gew.-%, wobei das kationische Polymer derart gewählt ist, dass das Viskositätsverhältnis Vs, definiert als Verhältnis zwischen der maximalen Viskosität in Gegenwart eines Tensids und der Viskosität bei Abwesenheit des Tensids, wenigstens mehr als 2 beträgt.

2. Zusammensetzung gemäß Anspruch 1, **dadurch gekennzeichnet, dass** sie im Bereich von 0,05 bis 0,5 Gew.-% des kationischen Polymers aufweist.

3. Zusammensetzung gemäß einem der vorausgegangenen Ansprüche, **dadurch gekennzeichnet, dass** das anionische Tensid gewählt ist aus:
- den Alkylestersulfonaten mit der Formel R-CH(SO₃M)-COOR', in der R ein Alkylradikal mit C₈-C₂₀, R' ein Alkylradikal mit C₁-C₆ und M ein Alkali-Kation, substituiertes oder nicht substituiertes Ammonium oder ein Derivat von einem Alkanolamin darstellt;
- den Alkylsulfaten mit der Formel ROSO₃M, in der R ein Alkyl- oder Hydroxyalkylradikal mit C₁₀-C₂₄ darstellt, wobei M ein Wasserstoffatom oder ein Alkali-Kation, substituiertes oder nicht substituiertes Ammonium oder ein Derivat von einem Alkanolamin darstellt, sowie deren oxyalkylenierten Derivaten, welche durchschnittlich 0,5 bis 6 oxyalkylenierte Einheiten aufweisen;
- den Alkylamidsulfaten mit der Formel RCONHR'OSO₃M, in der R ein Alkylradikal mit C₂-C₂₂, R' ein Alkylradikal mit C₂-C₃ darstellt, wobei M ein Wasserstoffatom oder ein Alkali-Kation, substituiertes oder nicht substituiertes Ammonium oder ein Derivat von einem Alkanolamin darstellt, sowie deren oxyalkylenierten Derivaten, welche durchschnittlich 0,5 bis 60 oxyalkylenierte Einheiten aufweisen;
- den Salzen von gesättigten oder ungesättigten Fettsäuren mit C₈-C₂₄;
- den Alkylbenzolsulfonaten mit C₉-C₂₀, den primären oder sekundären Alkylsulfonaten mit C₈-C₂₂, den Alkylglycerolsulfonaten, den sulfonierten Polycarbonsäuren, den Sulfonaten von Paraffin, den N-Acyl-N-alkyltauraten, den Alkylphosphaten, den Alkylisethionaten, den Alkylsuccinamaten, den Alkylsulfosuccinaten, den Monoestern oder Diestern von Sulfosuccinaten, den N-Acylsarcosinaten, den Sulfaten von Alkylglykosiden, den Polyethoxycarboxylaten.

4. Zusammensetzung gemäß einem der vorausgegangenen Ansprüche, **dadurch gekennzeichnet, dass** sie wenigstens ein Co-Tensid aufweist.

5. Verwendung eines Gemischs, welches aufweist:
- im Bereich von 0,1 bis 0,55 Gew.-% wenigstens eines anionischen Tensids,
- im Bereich von 0,01 bis 2 Gew.-% wenigstens eines kationischen Tensids gewählt aus den Hydroxypropylguarhydroxypropyltrimoniumchloriden, wobei das kationische Polymer derart gewählt ist, dass das Viskositätsverhältnis Vs, definiert als Verhältnis zwischen der maximalen Viskosität in Gegenwart eines Tensids und der Viskosität bei Abwesenheit des Tensids, wenigstens mehr als 2 beträgt,
als Strukturiermittel in wässrigen kosmetischen Zusammensetzungen.
